(19) Europäisches Patentamt
European Patent Office
Office européen des brevets

(11) Publication number: **0 338 993**
**A1**

(12) # EUROPEAN PATENT APPLICATION

(21) Application number: **89810291.8**

(22) Date of filing: **18.04.89**

(51) Int. Cl.4: **C 07 D 495/14**
A 61 K 31/55
//(C07D495/14,333:00,249:00,
243:00)

(30) Priority: **21.04.88 US 184537**

(43) Date of publication of application:
**25.10.89 Bulletin 89/43**

(84) Designated Contracting States:
**AT BE CH DE ES FR GB GR IT LI LU NL SE**

(71) Applicant: **SANDOZ AG**
**Lichtstrasse 35**
**CH-4002 Basel (CH)**

(84) Designated Contracting States:
**BE CH ES FR GB GR IT LI LU NL SE**

(71) Applicant: **SANDOZ-PATENT-GMBH**
**Humboldtstrasse 3**
**D-7850 Lörrach (DE)**

(84) Designated Contracting States: **DE**

(71) Applicant: **SANDOZ-ERFINDUNGEN**
**Verwaltungsgesellschaft m.b.H.**
**Brunner Strasse 59**
**A-1235 Wien (AT)**

(84) Designated Contracting States: **AT**

(72) Inventor: **Cheon, Seung Hoon**
**120 Carteret Street**
**Glen Ridge, N.J. 07028 (US)**

**Houlihan, William Joseph**
**15 Raynold Road**
**Mountain Lakes, N.J. 07046 (US)**

Claims for the following Contracting States: ES + GR.

(54) **6-Aryl-substituted-4h-thieno[2,3-e][1,2,4]triazolo [3,4-c][1,4]diazepines.**

(57) The invention discloses compounds of the formula I

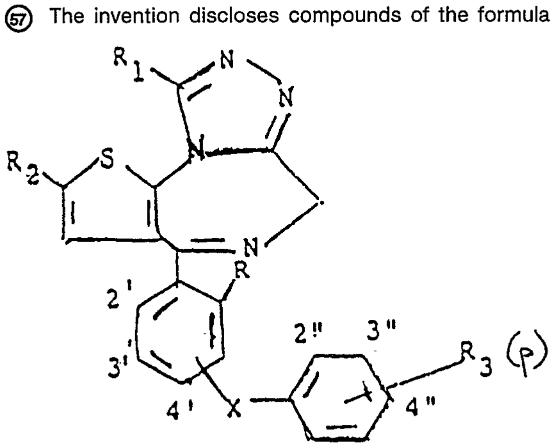

I

wherein the substituents have various significances.

They may be prepared by reaction of a diazepin-2-thione with an acethydrazide, by amidation,or by hydrolysis. They are indicated for use as inhibitors or platelet activating factor (PAF).

EP 0 338 993 A1

**Description**

## 6-ARYL-SUBSTITUTED-4H-THIENO[2,3-e][1,2,4]TRIAZOLO [3,4-c][1,4]DIAZEPINES

The present invention relates to 6-aryl-substituted-4H-thieno[2,3-e][1,2,4]triazolo[3,4-c] [1,4]diazepines and to their use as platelet activating factor (PAF) receptor antagonists and as inhibitors of PAF-induced blood platelet aggregation. The invention also relates to pharmaceutical compositions containing the afore-mentioned compounds as an active ingredient thereof and to the method of using such compositions for inhibiting PAF-mediated bronchoconstriction and extravasation, PAF induced hypotension, PAF-involved ischemic bowel disease and PAF-mediated, endotoxin-induced lung injury, for hyperreactive airways control induced by PAF or allergen, and for protection against endotoxin-induced hypotension and death.

WO 88/587 discloses 5-aryl- or 5-heteroaryl-substituted imidazo[2,1-a]isoquinolines exhibiting PAF-inhibiting activity.

The present invention concerns compounds of formula I

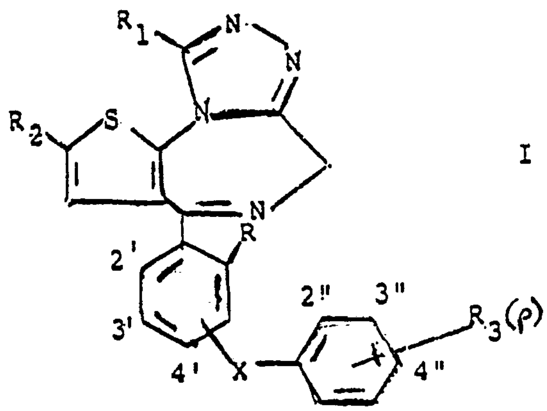

I

where
R is hydrogen or chloro;
$R_1$ is hydrogen; methyl or cyclopropyl;
$R_2$ is hydrogen; methyl; ethyl; a group of the formula

$-(CH_2)_m-\overset{O}{\overset{\|}{C}}-OR_4$ , where

m is 1 to 4 and $R_4$ is methyl, ethyl or an alkali metal cation; a group of the formula

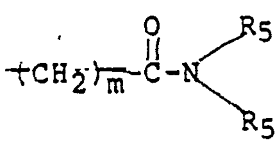 ,

where
m is as defined above and each $R_5$, independently, is straight or branched chain $C_{1-3}$alkyl, or the two $R_5$'s together with the nitrogen atom to which they are attached form a group of the formula

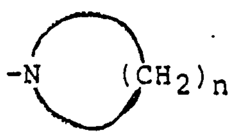 ,

where n
is 4 or 5, or a group of the formula

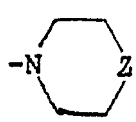 ,

where Z is
-O-, -S- or $-NCH_3-$; or a group of the formula

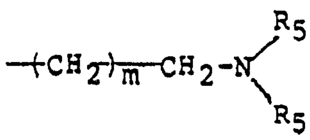 ,

where
m and the $R_5$'s are as defined above;
X is $-(CH_2)_m-$ where m is as defined above; $-OCH_2-$ or $-CH_2OCH_2-$;
p is 0 or an integer 1 to 3;
and
$R_3$ is chloro; fluoro; methyl; t-butyl; $OR_6$, where $R_6$ is methyl or ethyl; or a group

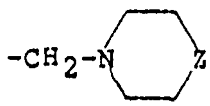 ,

where
Z is as defined above;
with the provisos that

1) when R is hydrogen, then X is in the 3′ or 4′ position, and when R is chloro, then X is in the 4′ position,
2) when $R_3$ is chloro, fluoro or methyl, then p is other than 3,
3) when $R_3$ is t-butyl or a group

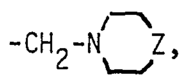

then p is 1, and
4) when p is 2, then the two $R_3$'s are not simultaneously in the 2″ and 6″ positions, in free form or in acid addition salt form where such salts exist.

The provisos are meant to exclude compounds which it is believed may not be readily prepared.

R preferably is hydrogen, $R_1$ preferably is methyl. $R_2$ preferably is hydrogen, $-(CH_2)_mCOOR_4$ or $-(CH_2)_mCON(R_5)_2$, especially hydrogen.

X preferably is $-(CH_2)_m-$. It preferably is in the 4′ position. p preferably is 3. $R_3$ preferably is chloro, fluoro or $-OR_6$. When p is 1 then $R_3$ preferably is in the 2″ or 4″, especially in the 4″ position. When p is 2 then the two $R_3$s preferably are in the 2″ and 4″ or in the 3″ and 4″ positions, especially in the 3″ and 4″ positions. When p is 3 then the three $R_3$s preferably are in the 3″, 4″ and 5″ positions.

m preferably is 2. $R_4$ preferably is methyl or an alkali metal cation, especially methyl. The two $R_5$s preferably are methyl or together with the nitrogen atom to which they are attached form a group of formula

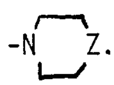

n preferably is 4. Z preferably is -O-. $R_6$ preferably is methyl.

A group of compounds of formula I is the compounds of formula I′:

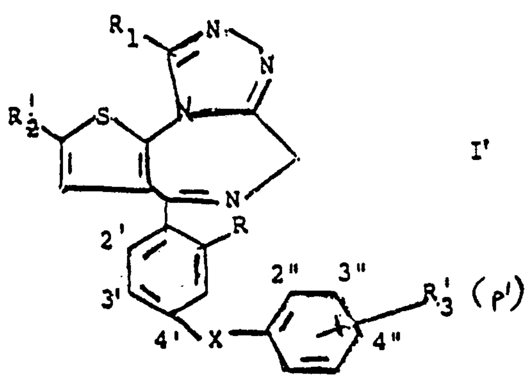

I′

where $R_2'$ is hydrogen; a group of the formula

$-(CH_2)_m-\overset{O}{\overset{\|}{C}}-OR_4'$ , where
$R_4'$ is methyl, sodium or potassium and m is as defined above;

or a group of the formula

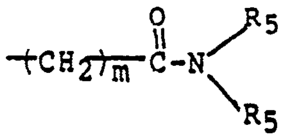,

where
m and the $R_5$'s are as defined above;
p' is an integer 1 to 3;
$R_3'$ is $OR_6$, where $R_6$ is as defined above;
or a group

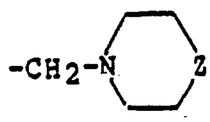,

where
Z is as defined above;
and R, $R_1$ and X are as defined above;
with the provisos that: 1) when $R_3'$ is a group

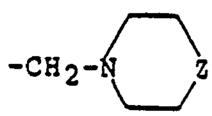,

then
p' is 1; and 2) when p' is 2, then the two $R'_3$s are not simultaneously in the 2'' and 6'' positions, in free form or in acid addition salt form where such salts exist.

A further group of compounds of formula I is the compounds of formula I'':

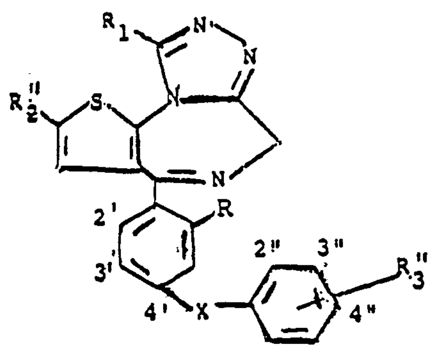

I''

where $R_2''$ is hydrogen; a group of the formula

$-(CH_2)_m-\overset{O}{\overset{\|}{C}}-OR'_4$, where
m and $R'_4$ are as defined above;
or a group of the formula

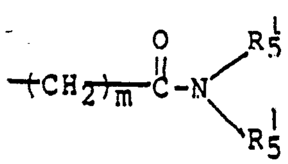,

where
m is as defined above and the two $R_5'$s together with the nitrogen atom to which they are attached form a group of the formula

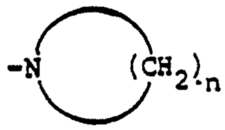 ,

where
n is as defined above, or a group of the formula

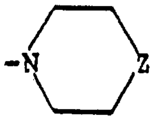 ,

where
Z is as defined above;
$R_3''$ is $OR_6$, where $R_6$ is as defined above;
and R, $R_1$, X and p′ are as defined above,
with the proviso that
when p′ is 2,
then the two $R_3''$'s are not simultaneously in the 2″ and 6″ positions, in free form or in acid addition salt form where such salts exist.

A further group of compounds of formula I is the compounds of formula I‴:

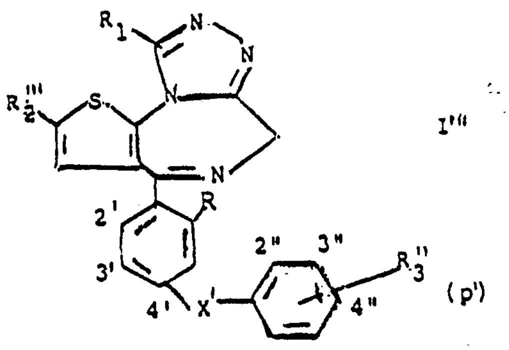

I‴

where $R_2'''$ is hydrogen; a group of the formula

$-(CH_2)_2-\overset{O}{\overset{\|}{C}}-OR'_4$ , where
R is as defined above;
or a group of the formula

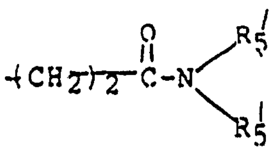 ,

where
the $R_5'$'s are as defined above;
X′ is $-(CH_2)_m-$ , where m is as defined above;
and R, $R_1$, p′ and $R_3''$ are as defined above,
with the proviso that
when p′ is 2,
then the two $R_3''$s are not simultaneously in the 2″ and 6″ positions, in free form or in acid addition salt form where such salts exist.

A further group of compounds of formula I is the compounds of formula $I^{iv}$

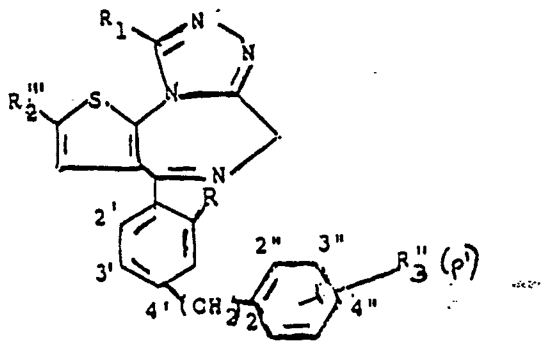

$I^{iv}$

where R, $R_1$,$R_2'''$ , p' and $R_3''$ are as defined above, with the proviso that when p' is 2, then the $R_3''$ 's aren't simultaneously in the 2″ and 6″ positions, in free form or in acid addition salt form where such salts exist.

A further group of compounds of formula I is the compounds of formula Is

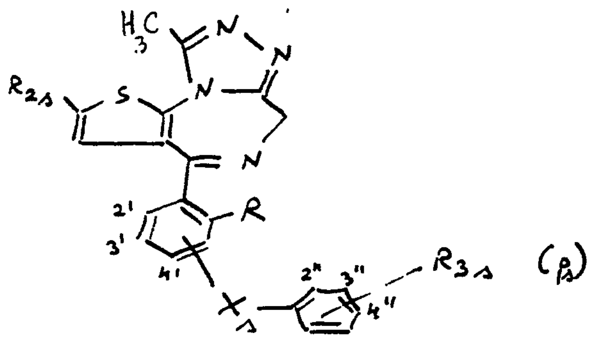

Is

wherein
R is as defined above,
$R_{2s}$ is hydrogen; a group of the formula $-(CH_2)_mCOOR_4$ wherein m and $R_4$ are as defined above; or a group of the formula

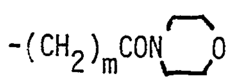

wherein m is as defined above,
$X_s$ is in the 3′ or 4′ position and is $-(CH_2)_m-$ wherein m is as defined above,
$p_s$ is 3, and
$R_{3s}$ is $-OR_6$ wherein $R_6$ is as defined above,
in free form or in acid addition salt form where such salts exist.

In the compounds of formula Is m preferably is 2.

A further group of compounds of formula I is the compounds of formula I wherein the substituents are as defined above with the proviso that $R_4$ is other than an alkali metal cation.

A compound of formula I may be obtained by a process comprising

a) when $R_2$ is hydrogen; methyl;ethyl; a group of the formula $-(CH_2)_mCOOR_4$ wherein $R_4$ is methyl or ethyl; or a group of the formula $-(CH_2)_mCH_2N(R_5)_2$,
reacting a corresponding compound of the formula A

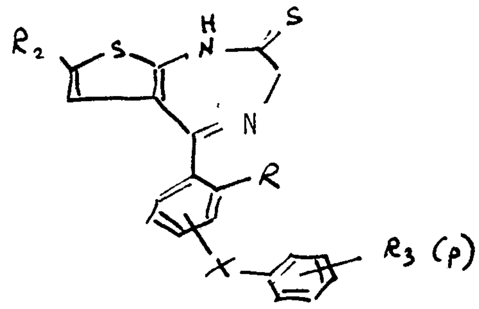

A

wherein the substituents are as defined above, with the proviso that $R_2$ is other than a group of the formula-$(CH_2)_mCON(R_5)_2$
wherein $R_2$ and m are as defined above,
with a corresponding compound of the formula AA
$R_1$-CONH-$NH_2$ AA
wherein $R_1$ is as defined above;
b) when $R_2$ is a group of the formula -$(CH_2)_mCON(R_5)_2$,
reacting a corresponding compound of formula I wherein $R_2$ is a group of the formula -$(CH_2)_mCOOR_4$ wherein m is as defined above and $R_4$ is methyl or ethyl,
with a corresponding compound of the formula B
$HN(R_5)_2$ B
wherein $R_5$ is as defined above; or
c) when $R_2$ is a group of the formula -$(CH_2)_mCOOR_4$ wherein $R_4$ is an alkali metal cation,
hydrolysing a corresponding compound of the formula I wherein $R_2$ is a group of the formula -$(CH_2)_mCOOR_4$ wherein $R_4$ is methyl or ethyl,
and recovering the resultant compound of the formula I in free form or in acid addition salt form where such salts exist.

The above process variants can be effected in a manner analogous to known methods.

Process variant a) is the reaction of a diazepine-2-thione compound with a hydrazide compound to yield a triazolodiazepine.

The reaction is typically carried out in an inert, organic solvent, e.g., an aromatic hydrocarbon such as benzene or toluene, or preferably in diglyme or another lower alkoxyethyl ether. As to reaction conditions, the reaction is conducted at a temperature of from 90° to 130°C, for example.

Process variant b) is the reaction of an ester with a secondary amine to yield an amide. The reaction is generally carried out at a temperature of from about 150° to about 190°C.

Process variant c) is the hydrolysis of an ester to an anionic salt. It is preferably effected under basis conditions, e.g. in an aqueous solution of an alkali metal hydroxide, preferably sodium or potassium hydroxide. The hydrolysis is optionally conducted in the presence of an inert, organic solvent such as a lower alkanol,e.g. methanol or ethanol, at a temperature of from about -10° to about 30°C.

The starting materials can be obtained in accordance with known methods, e.g. as described in the following reaction schemes, whereby the starting materials for process variant a) are represented for the compounds of formula A by the compounds of the formulae X and XX

STEP 1

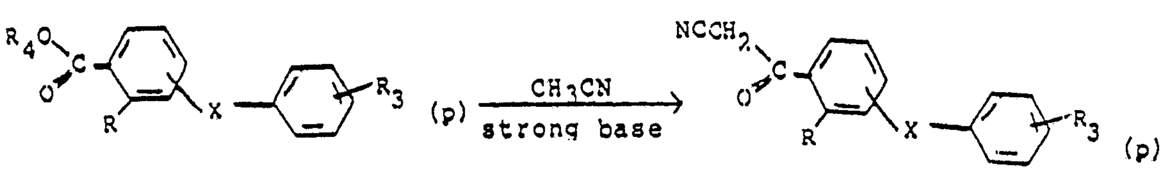


(II) (III)

where $R_4$ is methyl or ethyl, and R, X, p and $R_3$ are as defined above.

STEP 2

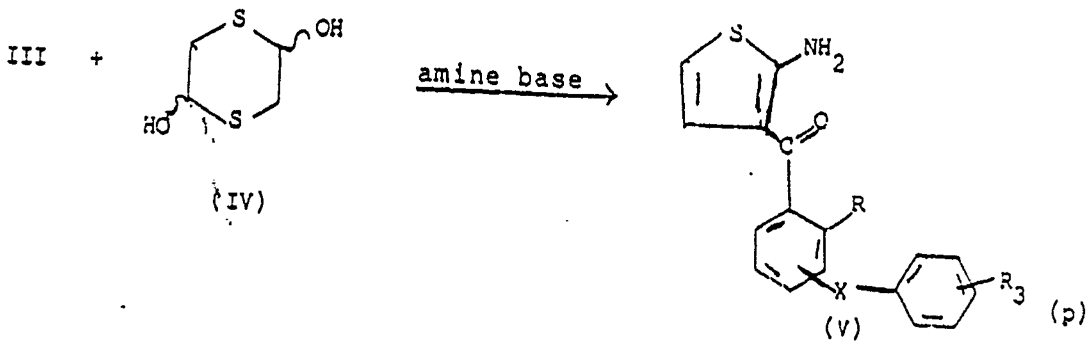


III + (IV) → (V)

where R, X, p and $R_3$ are as defined above.

STEP 3

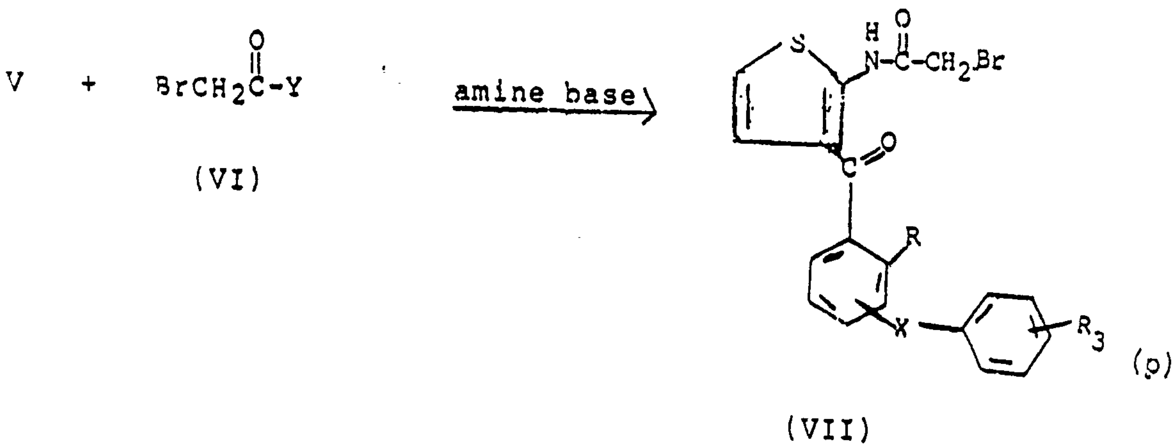


V + (VI) → (VII)

where Y is chloro or bromo and R, X, p and $R_3$ are as defined above.

STEP 4

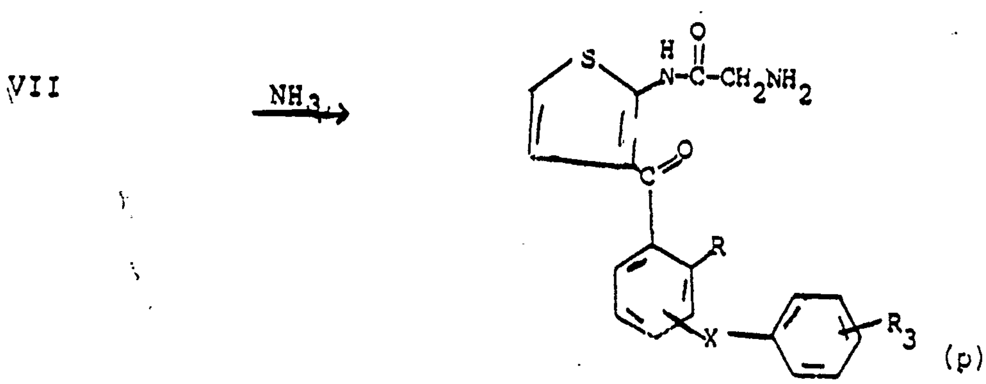


(VIII)

where R, X, p and $R_3$ are as defined above.

STEP 5

VIII

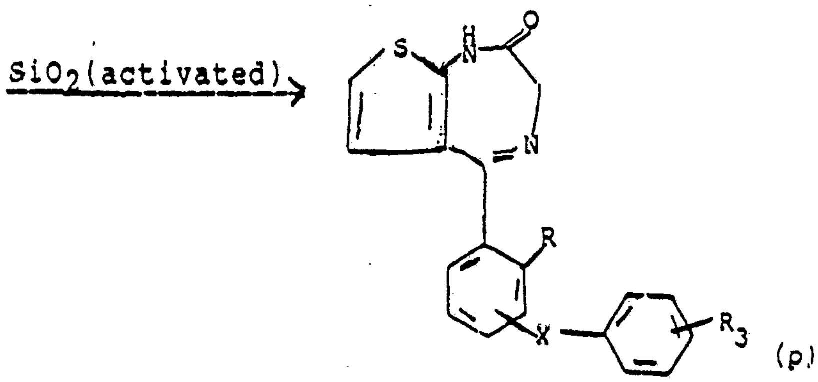


(IX)

where R, X, p and $R_3$ are as defined above.

STEP 6

IX

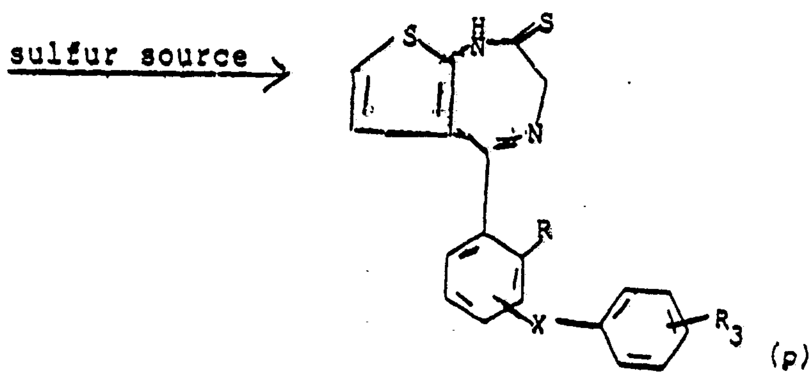


(X)

where R, X, p and $R_3$ are as defined above.

With respect to the individual steps:

Step 1 is typically carried out in an inert, organic solvent, e.g. a di-lower alkyl ether such as methyl t-butyl ether or a cyclic ether such as tetrahydrofuran, a polar, aprotic solvent such as dimethylformamide, or a mixture of a cyclic ether and a polar, aprotic solvent, e.g., a mixture of tetrahydrofuran and dimethylformamide. As to reaction conditions, the reaction is typically carried out at a temperature of from 30° to 75°C.

Step 2 is generally carried out in a polar, aprotic solvent such as dimethylformamide or dimethylacetamide at a temperature of from 25° to 75°C

As to Step 3 it is usually carried out in an inert, organic solvent, e.g., an aromatic hydrocarbon such as toluene or benzene, a halogenated, aliphatic hydrocarbon such as methylene chloride or chloroform, or a cyclic ether such as tetrahydrofuran. As to reaction conditions, the reaction is generally carried out at a temperature of from 0° to 35°C.

In Step 4, the reaction is typically conducted in an inert, organic solvent, e.g., a di-lower alkyl ether such as methyl t-butyl ether, a lower alkanol such as methanol, a cyclic ether such as tetrahydrofuran, or a mixture of a lower alkanol and a cyclic ether, e.g., a mixture of methanol and tetrahydrofuran. As regards reaction conditions, the reaction is normally conducted at a temperature of from 0 ° to 35°C.

Step 5 is typically conducted in an inert organic solvent, e.g., an aromatic hydrocarbon such as toluene, benzene or xylene, or a cyclic hydrocarbon such as cyclohexane, at a temperature of from 60° to 150°C

Step 6 is usually carried out in a solvent such as pyridine, N-methylpyridine or quinoline, or in an aromatic hydrocarbon such as benzene or toluene at a temperature of from 80° to 120°C

REACTION A

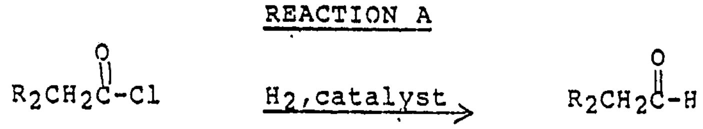


(XIII) (XIV)

where $R_2$ is methyl, ethyl, a group of the formula

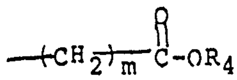

, or a group of the formula

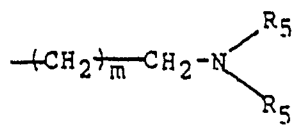

REACTION B

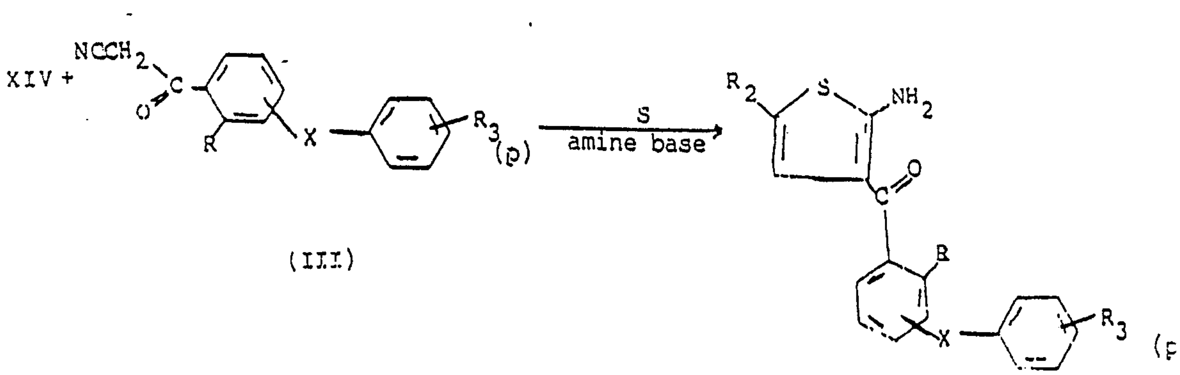


(III)

(XV)

where $R_2$ is as set forth in Reaction A, and R, X, p and $R_3$ are as defined above.

REACTION C

XV +

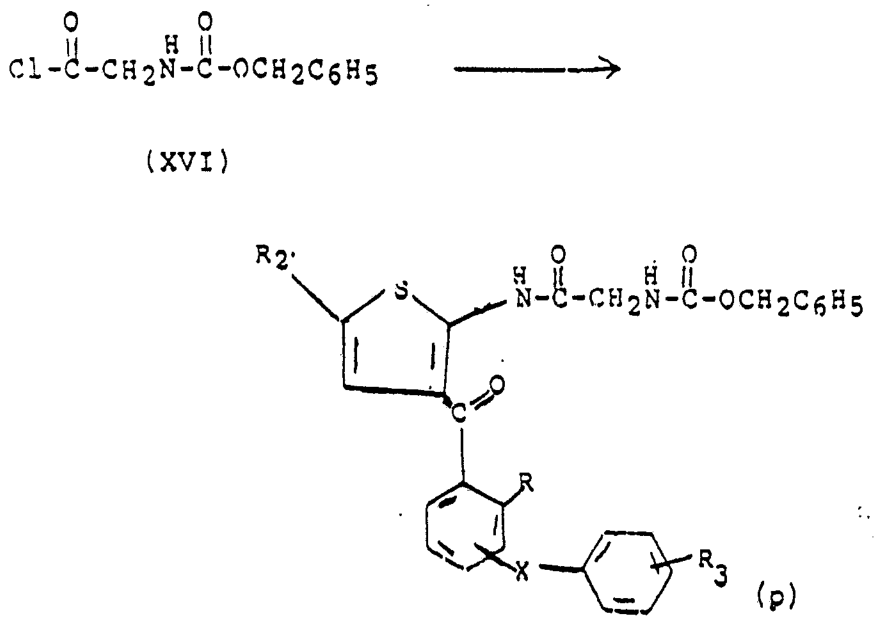

(XVI)

(XVII)

where $R_2$ is as set forth in Reaction A, and R, X, p and $R_3$ are as defined above.

REACTION D

XVII

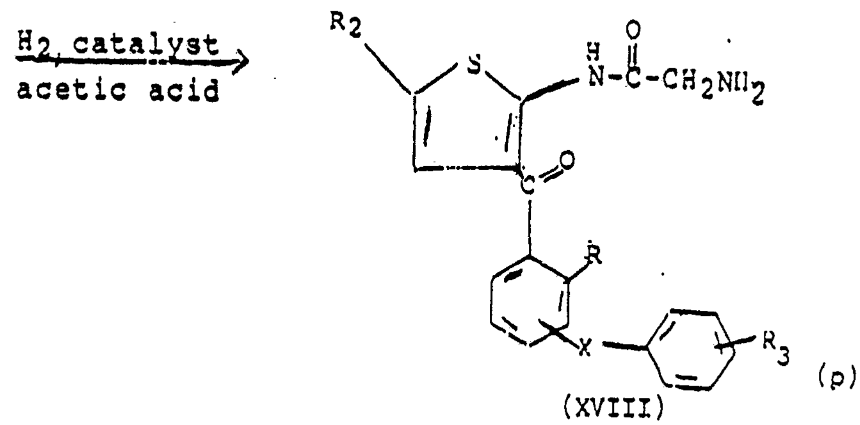


(XVIII)

where $R_2$ is as set forth in Reaction A, and R, X, p and $R_3$ are as defined above.

REACTION E

XVIII 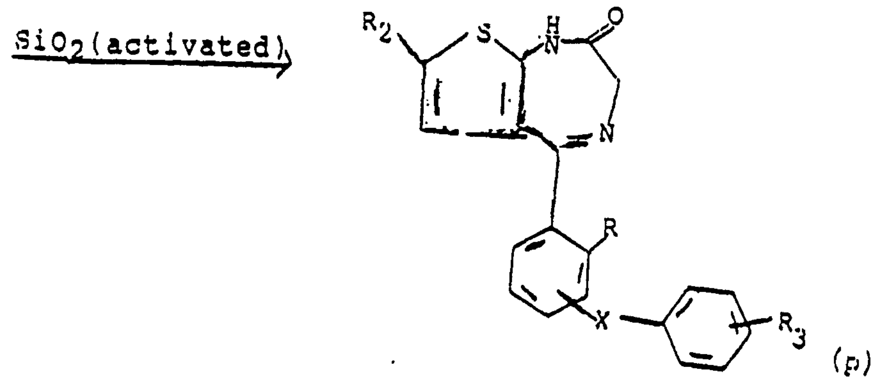


(XIX)

where $R_2$ is as set forth in Reaction A, and R, X, p and $R_3$ are as defined above.

REACTION F

XIX 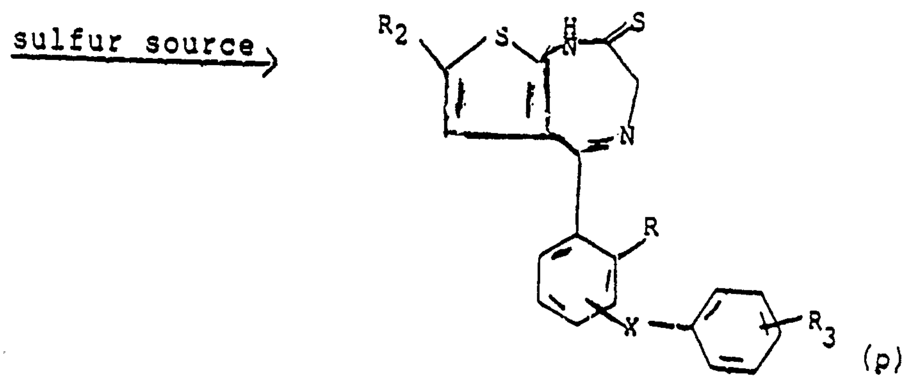


(XX)

where $R_2$ is as set forth in Reaction A, and R, X, p and $R_3$ are as defined above.

As to the reactions individually:

Reaction A is typically carried out by dissolving the acid chloride compound in an inert, organic solvent, e.g., a cyclic ether such as tetrahydrofuran or dioxane, adding a catalyst such as palladium or platinum on carbon and a mono- or di-lower alkyl pyridine, e.g., 2,6-lutidine (as a poison for the catalyst), and subjecting the resultant mixture to an elevated pressure of hydrogen gas at a temperature of from 15° to 25°C

Reaction B is typically carried out in a polar, aprotic solvent such as dimethylformamide and in the presence of an amine base such as triethylamine or N-methylpyrrolidine. The reaction temperature is typically from about 50°C to about 100°C.

In Reaction C a compound of the formula XVI is prepared by the addition of phosphorus pentachloride to a suspension of carbobenzyloxyglycin in dry ether and stirring the resultant mixture at ambient temperatures to yield a compound of formula XVII. The subsequent reaction is typically carried out in an inert, organic solvent, e.g., a halogenated, aliphatic hydrocarbon such as methylene chloride or chloroform, at a temperature of from 10° to 30°C

Reaction D involves dissolving a compound of formula XVII in glacial acetic acid, adding either palladium or platinum on carbon, and subjecting the resultant mixture to an elevated pressure of hydrogen gas at a temperature of from 15° to 25°C.

In Reaction E a compound of formula XVIII is treated with activated silica. The reaction conditions are analogous to those set forth above in Step 5.

Reaction F involves subjecting a compound of formula XIX to a sulfur source, e.g., phosphorus pentasulfide or Lawesson's Reagent (2,4-bis[4-methoxyphenyl]-1,3-dithia-2,4-diphosphetane-2,4-disulfide). The conversion

is carried out under the analogous conditions set forth above in Step 6.

The compounds of formula II, IV, VI, XI, XIII, XVI and XXII are either known or may be obtained by methods described in the literature, or may be obtained by methods analogous to those described in the literature.

Examples of pharmaceutically acceptable acid addition salts include those with mineral acids such as hydrochloric, hydrobromic, phosphoric and sulfuric acids, as well as those with organic acids such as tartaric, acetic, citric, malic, maleic, methanesulfonic and gluconic acids.

Final products and intermediates may be isolated and purified in conventional manner. Intermediates, where appropriate may be employed directly in the following step without purification.

The compounds of formula I, as well as their pharmaceutically acceptable acid addition salts, are useful as platelet activating factor inhibitors as indicated by their ability to inhibit platelet activating factor (PAF)-induced human platelet aggregation in vitro according to the Platelet Aggregation Inhibition Assay test (PAIA test) as follows:

Human subjects are kept aspirin free for one week and fasted overnight. Platelet rich plasma (PRP) is prepared by centrifugation (200 x g) of freshly drawn blood, anti-coagulated with 0.38% sodium citrate (final concentration). Platelet count is adjusted to 250 000 per µl using platelet poor plasma (ppp) obtained by a second centrifugation (700 x g) of the blood sample. An aliquot (0.38 ml) of the PRP is dispensed into cuvettes and maintained at room temperature (22° C) until used (but for not more than two hours). The PRP-containing cuvettes are incubated at 37° C and stirred at 900 rpm within a Payton Aggregometer which is activated to follow the light deflection pattern prior to the addition of the test compound. The test compound (dissolved in a suitable solvent mixture which does not influence platelet aggregation) is then added to a PRP-containing cuvette in an amount sufficient to provide a final concentration of 100µM. Between one and two minutes after the addition of the test compound, the aggregation inducing agent (PAF), dissolved in a buffer consisting of 0.01 M Tris-Tyrodes buffer with 0.25% bovine serum albumin (pH 7.4), is added to the PRP-containing cuvettes in an amount pre-determined to give a consistent aggregation response (either 0.1µM or 0.01µM). All aggregations are allowed to proceed for 6 minutes from the addition of the inducing agent. The aggregation response is quantitated by determining the area under the curve (AUC). The AUC calculated for the inducing agent alone is considered to be one hundred percent. The potential percent inhibition of the aggregation response is determined by dividing the AUC generated in the presence of the compound by the AUC of the inducing agent alone, multiplying by 100 and then subtracting from 100. The compounds demonstrating greater than 50% inhibition at 100µM are evaluated at lower concentrations to generate an $IC_{50}$ (50% inhibitory concentration) value.

Moreover, it has been found that the compounds of formula I are useful as platelet activating factor receptor antagonists as indicated by their ability to inhibit specific binding of ($^3$H)-PAF to platelets according to the Human Platelet PAF Receptor Assay test (Test A) as described in WO 88/587.

Furthermore, in view of their usefulness as PAF receptor antagonists, the compounds of formula I have been found useful as inhibitors of PAF-mediated broncho-constriction, which property was evaluated by the PAF-induced Pulmonary Inflation Pressure (PIP) Increase test (Test B) as described in WO 88/587.

Still further, the compounds of formula I are useful as inhibitors of PAF-mediated extravasation (the extrusion of plasma from the lumen of the blood vessels into the vessel well and surrounding tissues) measured as a function of hemoconcentration according to the PAF-induced Extravasation test (Text C) described in WO 88/587. From the values obtained an $ED_{50}$ is generated.

Yet still further, the compounds of formula I are useful as inhibitors of PAF-induced hypotension as measured by their ability to inhibit the lowering of blood pressure levels induced by PAF according to the following test (Test D):

Male Wistar rats, weighing approximately 300 g, are anesthetized and their carotid arteries cannulated to enable their diastolic and systolic arterial blood pressure measurements to be recorded. PAF is then administered intravenously at either 100 or 500 ng/kg, and the blood pressure drop (within 10 sec) and recovery time required to reach the pre-injection blood pressure level are measured. At 100 ng/kg, a 30% decrease in blood pressure and a 3 to 4 minute recovery time are observed, whereas at 500 ng/kb, a 52% decrease in blood pressure and a 10 minute recovery time are observed. In order to measure the effectiveness of a compound for both the inhibition of blood pressure decreases and shortening of the recovery time, the test compound is administered intravenously over a range of between 5 and 7 dosage levels (1 or 2 test animals per dose) and between 1 and 5 minutes prior to the introduction of PAF to generate an $ED_{50}$.

Yet even still further, the compounds of formula I are useful as inhibitors of PAF-induced ischemic intestinal necrosis, which property can be measured in accordance with the following test (Test E):

Following essentially the procedure of F. Gonzales-Crussi and W. Hsueh published in J. Amer. Pathol., 112, p. 127-135 (1983), male Sprague-Dawley rats, weighing approximately between 260 and 300g, are anesthetized and their carotid arteries cannulated and connected to a blood pressure transducer and recorder. The test compound is introduced into a cannula inserted into the jugular vein at a time 10 minutes prior to the administration of PAF. The abdomen is then incised along the midline and 2µg of PAF or 20µg of LPS (lipopolysaccharide) immediately followed by 1µg of PAF are injected into the abdominal aorta at the level of the renal artery. The abdominal incision is then covered with saline-moistened gauze and the intestine exposed and examined periodically up to 2 to 3 hours prior to sacrifice. Into the jugular vein is then injected 5 ml of 2% Evans Blue to assess the degree of intestinal perfusion. Blocks of intestinal tissue are then taken for microscopic examination to determine either the extent of necrosis or to verify the absence of necrosis when

inhibited by the test compound. Microscopic changes in the intestine are assessed by hematoxylin and eosin staining. The test compound is assessed for its ability to alleviate or prevent the development of gross and microscopic lesions and may be expressed in terms of the number of animals in which inhibition is observed relative to the control (taken to be 100%).

Yet even still further more, the compounds of formula I are useful as inhibitors of PAF-mediated, endotoxin-induced lung injury and, analogously, endotoxin-induced-septic shock and adult respiratory distress syndrome. Test methods for these three indications are described in WO 88/587.

Yet even still further even more, the compounds of formula I are useful in controlling hyperreactive airways induced by PAF or allergen, which property can be measured in accordance with the following procedure (Test G):

Male Hartley guinea pigs weighing 250gm are sensitized to ovalbumin by aerosol inhalation exposure. The test animals are then subsequently rechallenged with ovalbumin aerosol repeatedly (3 to 6 times) over a period of two to three weeks. Airway reactivity is assessed by an acetylcholine dose response curve at times (1 to 3 days) after the last ovalbumin exposure. The test compound is assessed for its ability to control hyperreactivity airways by administering it orally with a gavage tube in an acceptable vehicle prior to each ovalbumin antigen exposure.

Yet even more still further even more, the compounds of formula I are useful in protecting against endotoxin-induced hypotension, which property can be measured according to the following procedure (Test H):

Male Sprague-Dawley rats weighing between 250 and 270gm are anesthetized with sodium pentobarbital (50mg/kg i.p.) and the left common carotid artery is cannulated (PE-50 tubing) and connected to a P50 pressure transducer. Mean arterial pressures and diastolic and systolic measurements are recorded using a Gould 2400S physiograph. Blood flow of the mesenteric artery is measured on a calibrated electromagnetic flowmeter probe. Blood is collected via the femoral artery into heparinized capillary tubes and centrifuged to determine hematocrit values.

Endotoxin from E.coli serotype 0111:$B_4$ is prepared fresh daily and administered by i.v. injection to the test animals in tris-Tyrode's buffer over a 1 to 50 mg/kg dosage range to establish a dose-response profile. The administration of endotoxin at 15mg/kg i.v. produced a $54 \pm 8\%$ decrease in mean arterial pressure and a corresponding 80% decrease in mesenteric artery blood flow. The test compound is assessed for its ability to protect against endotoxin-induced hypotension by administering it intravenously after endotoxin administration and measuring the recovery of blood pressure and mesenteric artery blood flow. The $ED_{50}$ value of the test compound is determined using linear regression fitting of inhibition profiles from 5 to 6 doses (3 animals per dose).

Lastly, the compounds of formula I are useful in protecting against endotoxin-induced death, which property can be measured according to the following procedure (Test I):

Healthy male BALB/c mice weighing between 24 and 27g. are allowed to acclimate for 1 week with access to food and water. The test animals are then placed in a ventilated plexiglass restrainer that allows access to the tails. After the tails are allowed to immerse in warm water ( 38°C) for 30 seconds, endotoxin from E. coli serotype 0111:$B_4$ is administered in a single injection at 2ml/kg body weight to produce lethality at the desired effect of LD 70-90. The test compound is assessed for its ability to protect against endotoxin-induced death by administering it orally in a single bolus at a volume of 1ml/kg body weight. Each treatment group consists of 7 to 10 test animals, every dosage is considered as a separate group and control groups are dosed with vehicles (water tris-Tyrodes's buffer, 1% CMC, etc) only. The percent mortality (or survival) is expressed by the number of deaths (or survivors) within the observation period. Values obtained are mean and standard error of mean of a single treatment which represents multiple days results for reproducibility. The $ED_{50}$ value of the test compound is determined using a Students t test (2 tail) for significance.

The compounds of formula I, in free form or in pharmaceutically acceptable salt form, may be combined with one or more pharmaceutically acceptable carriers and, optionally, one or more other conventional pharmaceutical adjuvants and administered orally in the form of tablets, dispersible powders, granules, capsules, elixirs, suspensions and the like or parenterally in the form of sterile injectable solutions or suspensions. The compositions may be prepared by conventional means.

The precise dosage of a compound of formula I, or a pharmaceutically acceptable acid addition salt thereof, to be employed for inhibiting platelet activating factor (PAF) depends upon several factors including the host, the nature and the severity of the condition being treated, the mode of administration and the particular compound employed.

However, in general, satisfactory inhibition or antagonism of platelet activating factor, is achieved when a compound is administered orally at a daily dosage of 0.05-100, preferably 0.1-30 mg/kg body weight or, for most larger primates, a daily dosage of 1-500 mg, preferably 1-50 mg. A typical oral dosage is 5 mg, three times a day.

For the other uses mentioned above satisfactory activity is achieved when a compound is administered orally at a daily dosage of 0.2-100, preferably 0.2-50mg/kg body weight or, for most larger primates, a daily dosage of 100-2000mg, preferably 10-350mg. A typical oral dosage is 50 or 100mg, two or three times a day.

Examples of compositions are as follows:

| Ingredients | Weight (mg) | |
|---|---|---|
| | tablet | capsule |
| compound of formula I, e.g., the compound of Example 1 | 5 | 5 |
| tragacanth | 10 | - |
| lactose (spray-dried) | 257.5 | 95 |
| corn starch | 15 | - |
| talcum | 10 | - |
| magnesium stearate | 2.5 | - |
| Total | 300.0 | 100 |

| Ingredients | Weight (mg) | |
|---|---|---|
| | tablet | capsule |
| compound of formula I, e.g., the compound of Example 1 | 50 | 50 |
| tragacanth | 10 | - |
| lactose (spray-dried) | 212.5 | 100 |
| corn starch | 15 | - |
| talcum | 10 | - |
| magnesium stearate | 2.5 | - |
| Total | 300.0 | 100 |

Such compositions also form part of the invention.

The invention also includes a method for the preparation of a pharmaceutical composition comprising mixing a compound of the formula I in free form or in pharmaceutically acceptable acid addition salt form,with a a pharmaceutically acceptable carrier or diluent.

It also includes the use of such a compound for the preparation of a pharmaceutical composition comprising a compound of the formula I in free form or in pharmaceutically acceptable acid addition salt form.

It also includes the use of such a compound in the above indications, and a method of treatment which comprises administering a therapeutically effective amount of a compound of formula I in free form or in pharmaceutically acceptable acid addition salt form to a subject in need of such treatment.

It also includes the compounds of the formula I in free form or in pharmaceutically acceptable acid addition salt form for use as a pharmaceutical in particular for use as a PAF inhibitor, especially for use in the above indications.

The compound of Example 1 is the preferred compound. The following results were obtained in the above tests with this compound:

| | | |
|---|---|---|
| PAIA test: | $IC_{50}$ = | 12.0 μM |
| Test A: | $IC_{50}$ = | 0.22 μM |
| Test B: | $ED_{50}$ = | 0.8 mg/kg p.o. |
| Test C: | $ED_{50}$ = | 0.5 mg/kg p.o. |

The following Examples, in which all temperatures are in °C, illustrate the invention.

## EXAMPLE 1

6-[4-[2-(3,4,5-Trimethoxyphenyl)ethyl]phenyl]-4H-thieno [2,3-e][1,2,4]triazolo[3,4-c][1,4]diazepine
($R_1$ = Me; $R_2$ = H; $R_3$ = OMe in the 3″, 4″ and 5″ positions; X = $-(CH_2)_2-$in the 4′ position; R = H)

[process variant a)]

To a solution of 0.52g (1.1 mmol) of the compound prepared in f) below in 15ml of dry diglyme was added 0.426g (5.7 mmol) of acethydrazide, and the resultant mixture was heated to 110°C under a nitrogen atmosphere and maintained at this temperature for 4 hours. After cooling the reaction mixture to 25°C, the solvent was evaporated in vacuo and the resultant residue was diluted with methylene chloride, washed successively with water and brine, and dried over anhydrous magnesium sulfate. The solvent was then evaporated under reduced pressure to yield a crude product which was purified by column chromatography on silica gel employing a mixture of methylene chloride and methanol in a 20:1 ratio as the eluent to yield the title compound as a yellow solid (M.P. 264°C).

The starting material is obtained as follows:

a) Preparation of 3-oxo-3-[4-[2-(3,4,5-trimethoxyphenyl) ethyl]phenyl]propanenitrile

To a solution of 10g (30 mmol) of methyl 4-[2-(3,4,5-trimethoxyphenyl)ethyl]benzoate and 1.74ml (33 mmol) of acetonitrile in a mixture of 30ml of dry N,N-dimethylformamide and 20ml of dry tetrahydrofuran was added, portionwise under a nitrogen atmosphere at 0°C, 3g (76 mmol) of 60% suspension of sodium hydride in mineral oil. The resulting suspension was heated under a flow of nitrogen to 65°C and maintained at this temperature for 2 hours. After cooling to 25°C, the resultant mixture was quenched with 3ml of methanol and the solvent was removed in vacuo. The resultant residue was dissolved in 60ml of water, acidified with 2N hydrochloric acid to a pH between 4 and 5 and then extracted with ethyl acetate. The combined organic phase was then washed successively with water and saturated sodium chloride solution and dried over magnesium sulfate, after which time the solvent was removed under reduced pressure. The crude product was then purified on silica gel employing a mixture of ethyl acetate and hexane in a 1:1 ratio as the eluent to yield a white crystalline solid.

b) Preparation of 2-amino-3-thienyl-4-[2-(3,4,5-trimethoxyphenyl)ethyl]phenylmethanone

To a solution of 1.35g of the compound prepared in a) above in 8ml of dry N,N-dimethylformamide was added, successively and under a nitrogen atmosphere, 0.6g (4 mmol) of 1,4-dithiane-2,5-diol and 0.14ml (1 mmol) of triethylamine, and the resultant mixture was heated to 50°C and maintained at this temperature for 4 hours. After cooling the reaction mixture to 25°C., it was diluted with ethyl acetate and the resultant solution was washed successively with water and a saturated sodium chloride solution. The organic layer was dried over magnesium sulfate and evaporated under reduced pressure to yield a crude product which was purified on silica gel employing a mixture of ethyl acetate and hexane in a 1:1 ratio as the eluent to yield a yellow foam.

c) Preparation of 2-bromo-N-3-[4-[2-(3,4,5-trimethoxyphenyl)ethyl]benzoyl]thienylacetamide

To a solution of 1.3g of the compound prepared in b) above in 10ml of dry methylene chloride was added, successively and under a nitrogen atmosphere, 0.74ml (4.2 mmol) of N,N-diisopropylethylamine and 0.31ml (3.6 mmol) of bromoacetylbromide, and the resultant mixture was stirred at ambient temperature for 3 days. The reaction mixture was diluted with methylene chloride, washed successively with water and a saturated sodium chloride solution, and dried over magnesium sulfate. The solvent was then removed under reduced pressure to yield a crude product which was purified by column chromatography on silica gel employing a mixture of ethyl acetate and hexane in a 1:1 ratio as the eluent to yield a yellow foam.

d) Preparation of 2-amino-N-3-[4-[2-(3,4,5-trimethoxyphenyl)ethyl]benzoyl]thienylacetamide

Dry ammonia gas was bubbled into a solution of 1g of the compound prepared in c) above in a mixture of 10ml of dry methanol and 10ml of dry tetrahydrofuran for 5 minutes, and the resultant mixture was stirred at ambient temperature for 16 hours in a closed system. The solvent was evaporated in vacuo and the crude residue was purified by column chromatography on silica gel employing a mixture of ethyl acetate and hexane in increasing amounts of the latter, the initial ratio being 1:1 by volume, with elution taking place when the ratio became 1:5 by volume and resulted in a yellow foam.

e) Preparation of
1,3-dihydro-5-[4-[2-(3,4,5-trimethoxyphenyl)ethyl]phenyl]-2H-thieno[2,3-e][1,4]diazepin-2-one

To a solution of 0.66g (1.45 mmol) of the compound prepared in d) above in 15ml of dry toluene was added 1.5g of silica gel (70-230 mesh), and the resultant mixture was heated to reflux and maintained at reflux temperature for 2 hours using a Dean-Stark trap. After cooling the reaction mixture to 25°C, it was filtered and the silica gel was washed with hot methanol. The combined organic phase was then evaporated to yield a yellow foam.

f) Preparation of
1,3-dihydro-5-[4-[2-(3,4,5-trimethoxyphenyl)ethyl]phenyl]-2H-thieno[2,3-e][1,4]diazepin-2-thione

To a solution of 0.6g (1.4 mmol) of the compound prepared in e) above in 30ml of dry pyridine was added 0.37g (0.82 mmol) of phosphorus pentasulfide, and the resultant mixture was heated to 90°C and maintained

at this temperature for 30 minutes under a nitrogen atmosphere. After cooling the reaction mixture to 25° C, the solvent was evaporated and the resultant residue was diluted with methylene chloride. The organic phase was then washed successively with water and brine, dried over anhydrous magnesium sulfate and evaporated to yield a red foam.

## EXAMPLE 2

9-Methyl-4-[4-[2-(3,4,5-trimethoxyphenyl)ethyl]phenyl-6H-thieno[3,2-f][1,2,4]triazolo[4,3-a][1,4]diazepine-2-propanoic acid, methyl ester
($R_1$ = Me; $R_2$ = $-(CH_2)_2COOMe$; $R_3$ = OMe in the 3″, 4″ and 5″ positions; X = $-(CH_2)_2-$ in the 4′ position; R = H)

[process variant a)]
The title compound (M.P. 78-80° C) is obtained as a yellow solid in a manner analogous to Example 1, using in place of the compound prepared in Example 1f), an approximately equivalent amount of the compound prepared in f) below.
The starting material is obtained as follows:

a) Preparation of 1-oxopentanoic acid, methyl ester
To a solution of 8.23g (50 mmol) of methyl 4-(chloroformyl)butyrate in 200ml of tetrahydrofuran were added 5.4g (50 mmol) of 2,6-lutidine and 0.75g of 10% palladium on carbon, and the resultant mixture was then hydrogenated under a 41 p.s.i. atmosphere of hydrogen gas (Parr Shaker) for 3 hours. The reaction mixture was then filtered and the filtrate was evaporated under reduced pressure to obtain an oil which was washed with diethyl ether and then filtered. The resultant filtrate was then evaporated to yield a clear liquid.

b) Preparation of 5-amino-4-[4-[2-(3,4,5-trimethoxyphenyl)ethyl]benzoyl]-2-thiophene propanoic acid, methyl ester
A mixture of 1.53g (12 mmol) of the compound prepared in a) above, 4g (12 mmol) of the compound prepared in a) of Example 1 and 0.42g of sulfur in 10ml of N,N-dimethylformamide was heated to 70° C and maintained at this temperature for 1 hour, after which time 1ml of triethylamine was added to the mixture. The resultant mixture was stirred at 70° C for an additional 30 minutes, and then the reaction mixture was cooled to ambient temperature. The solvent was then evaporated and the resultant residue was diluted with ethyl acetate. The resultant solution was then washed successively with water and brine, dried over magnesium sulfate and filtered. The solvent was then removed under reduced pressure and the crude product was purified by column chromatography on silica gel employing a mixture of ethyl acetate and hexane in a 1:1 ratio as the eluent to yield a brown gum.

c) Preparation of 5-[2-[1-oxo-2-(phenylmethoxycarbonylamino)ethyl]amino]-4-[4-[2-(3,4,5-trimethoxyphenyl) ethyl]benzoyl]-2-thiophenepropanoic acid, methyl ester
To a suspension of 2.24g (11 mmol) of carbobenzyloxyglycine in 60ml of dry ether was added, under a nitrogen atmosphere, 1.75g (8.4mmol) of phosphorus pentachloride, and the resultant mixture was stirred for 1 hour at room temperature. The resultant solution was then added to a solution of 3.7g (7.7 mmol) of the compound prepared in b) above in 30ml of chloroform via cannular. The reaction mixture was then stirred for 16 hours at room temperature, diluted with methylene chloride, and the resultant solution was washed successively with water, a saturated sodium bicarbonate solution and brine. The organic layer was then dried over magnesium sulfate, filtered and the solvent was removed under reduced pressure. The crude product was then purified by column chromatography on silica gel employing a mixture of ethyl acetate and hexane in decreasing amounts of the latter, the initial ratio being 1:2 by volume, with elution taking place when the ratio became 1:1 by volume and resulted in a yellow foam.

d) Preparation of
5-[(2-amino-1-oxoethyl)amino]-4-[4-[2-(3,4,5-trimethoxyphenyl)ethyl]benzoyl]-2-thiophenepropanoic acid, methyl ester
To a solution of 2.2g (3.3 mmol) of the compound prepared in c) above in 100ml of glacial acetic acid was added 2.2g of 10% palladium on carbon, and the resultant mixture was then stirred under a hydrogen atmosphere for 16 hours. The reaction mixture was then filtered and the filtrate was evaporated under reduced pressure to obtain an oil which was dissolved in ethyl acetate. The resultant solution was then washed successively with water, a saturated sodium bicarbonate solution and brine. The organic layer was then dried over magnesium sulfate, filtered and the solvent was removed under reduced pressure to yield the desired product.

e) Preparation of 7,8-dihydro-7-oxo-4-[4-[2-(3,4,5-trimethoxyphenyl)ethyl]phenyl]-6H-thieno[2,3-e][1,4]-diazepine-2-propanoic acid, methyl ester

Following essentially the procedure of Example 1e), and using in place of the compound prepared in Example 1d), an approximately equivalent amount of the compound prepared in d) above, a yellow foam was obtained.

f) Preparation of 7,8-dihydro-7-thioxo-4-[4-[2-(3,4,5-trimethoxyphenyl)ethyl]phenyl]-6H-thieno[2,3-e][1,4]-diazepine-2-propanoic acid, methyl ester

Following essentially the procedure of Example 1f), and using in place of the compound prepared in Example 1e), an approximately equivalent amount of the compound prepared in e) above, a reddish orange foam was obtained.

## EXAMPLE 3

3-[9-Methyl-4-[4-[2-(3,4,5-trimethoxyphenyl)ethyl]phenyl]-6H-thieno[3,2-f][1,2,4]triazolo[4,3-a][1,4]diazepin-2-yl]-1-oxopropylmorpholine

($R_1$ = Me;

$R_2 = -(CH_2)_2CO$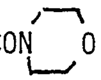;

$R_3$ = OMe in the 3″, 4″ and 5″ positions; X = $-(CH_2)_2-$ in the 4′ position; R = H)

[process variant b)]

A solution of 0.33g (0.59 mmol) of the compound of Example 2 in 3ml of dry morpholine was heated at 180°C in a sealed tube for 5 hours, and then cooled to room temperature. The solvent was evaporated and the resultant residue was purified by column chromatography on silica gel employing a mixture of methylene chloride and methanol in a 9:1 ratio as the eluent to yield the title compound as a tan solid (M.P. 105°-107°C).

## EXAMPLE 4

(8-Carboxyethyl-1-methyl-6-[4-[2-(3,4,5-trimethoxyphenyl)ethyl]phenyl]-4H-thieno(2,3-e][1,2,4]triazolo[3,4-c][1,4]diazepine, sodium salt

($R_1$ = Me; $R_2$ = $-(CH_2)_2COONa$; $R_3$ = OMe in the 3″, 4″ and 5″ positions; X = $-(CH_2)_2-$ in the 4′ position; R = H)

[process variant c]

To a solution of 0.06 g (0.11mmol) of the compound of Example 2 in 1 ml of ethanol was added, with stirring, a 1N solution of sodium hydroxide which was pre-cooled to 0°C. The resultant solution was then stirred for 30 minutes while the temperature was maintained at 0°C. Diethyl ether was then added and the resulting precipitate was filtered and dried to yield the title compound as a tan solid (M.P. 190°C).

## EXAMPLE 5

6-[2-Chloro-4-[2-(3,4,5-trimethoxyphenyl)ethyl]phenyl]-1-methyl-4H-thieno[2,3-e][1,2,4]triazolo[3,4-c][1,4]-diazepine

($R_1$ = Me; $R_2$ = H; $R_3$ = OMe in the 3″, 4″ and 5″ positions; X = $-(CH_2)_2-$ in the 4′ position; R = Cl)

[process variant a)]

The title compound is obtained in a manner analogous to Example 1,as a light brown solid (M.P. 178-180°C), using in place of the compound of Example 1f), an approximately equivalent amount of the compound prepared in f) below.

The starting material is obtained as follows:

a) Preparation of 2-chloro-β-oxo-4-[2-[3,4,5-trimethoxyphenyl)ethyl]benzenepropanenitrile

Following essentially the procedure of Example 1a), and using in place of methyl-4-[2-(3,4,5-trimethoxyphenyl)ethyl]benzoate, an approximately equivalent amount of 2-chloro-4-[2-(3,4,5-trimethoxyphenyl)ethyl]benzoic acid, methyl ester, a white solid was obtained.

b) Preparation of 2-amino-3-thienyl-2-chloro-4-[2-(3,4,5-trimethoxyphenyl)ethyl]methanone

Following essentially the procedure of Example 1b), and using in place of the compound prepared in Example 1a), an approximately equivalent amount of the compound prepared in a) above, a yellow oil was obtained.

c) Preparation of bromo-N-[3-[2-chloro-4-[2-(3,4,5-trimethoxyphenyl)ethyl]benzoyl]thien-2-yl]acetamide

To a solution of 0.57g of the compound prepared in b) above in 5ml of toluene was added, successively at ice water bath temperature, 0.64ml of pyridine and 0.34ml (3.9mmol) of bromoacetylbromide, and the resultant mixture was stirred at room temperature for 16 hours. The reaction mixture was diluted with ethyl acetate, washed successively with water and brine, and dried over magnesium sulfate. The solvent was evaporated to yield a brown oil.

d) Preparation of amino-N-[3-[2-chloro-4-[2-(3,4,5-trimethoxyphenyl)ethyl]benzoyl]thien-2-yl]acetamide

Following essentially the procedure of Example 1d), and using in place of the compound prepared in Example 1c), an approximately equivalent amount of the compound prepared in c) above, a yellow foam was obtained.

e) Preparation of
1,3-dihydro-5-[2-chloro-4-[2-(3,4,5-trimethoxyphenyl)ethyl]phenyl]-2H-thieno-[2,3-e][1,4]diazepin-2-one

Following essentially the procedure of Example 1e), and using in place of the compound prepared in Example 1d), an approximately equivalent amount of the compound prepared in d) above, a yellow foam was obtained.

f) Preparation of
1,3-dihydro-5-[2-chloro-4-[2-(3,4,5-trimethoxyphenyl)ethyl]phenyl]-2H-thieno-[2,3-e][1,4]diazepin-2-thione

To a solution of 0.25g (0.53 mmol) of the compound prepared in e) above in 5ml of toluene was added 0.13g (0.32mmol) of Lawesson's Reagent, and the resultant mixture was heated to 90°C and maintained at this temperature for 3 hours. After cooling the reaction mixture to 25°C, the solvent it was diluted with 50ml methylene chloride, washed successively with 1ml of water and 10ml of brine, dried over magnesium sulfate and evaporated. The crude residue was pruified by column chromatography on silica gel employing a mixture of ethyl acetate and hexane in a 2:1 ratio as the eluent to yield an orange foam.

## Claims

1. A compound of formula I:

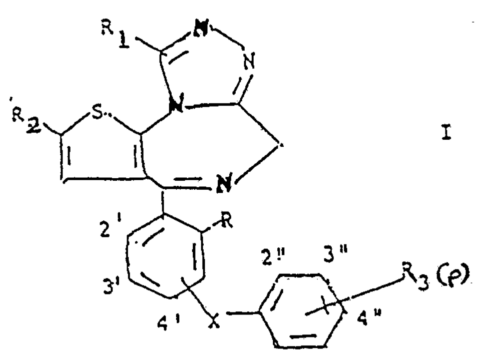

I

where
R is hydrogen or chloro;
$R_1$ is hydrogen; methyl or cyclopropyl;
$R_2$ is hydrogen; methyl; ethyl; a group of the formula

$$-(CH_2)_m-\overset{O}{\overset{\|}{C}}-OR_4$$ , where

m is 1 to 4 and $R_4$ is methyl, ethyl or an alkali metal cation; a group of the formula

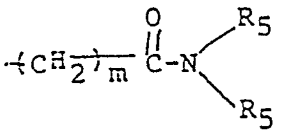,

where

m is as defined above and each $R_5$, independently, is straight or branched chain $C_{1-3}$alkyl, or the two $R_5$'s together with the nitrogen atom to which they are attached form a group of the formula

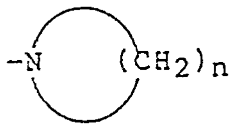,

where n

is 4 or 5, or a group of the formula

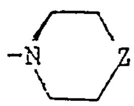,

where Z is

-O-, -S- or $-NCH_3-$; or a group of the formula

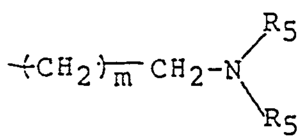,

where

m and the $R_5$'s are as defined above;

X is $-(CH_2)_{\overline{m}}$ where m is as defined above; $-OCH_2-$ or $-CH_2OCH_2-$;

p is 0 or an integer 1 to 3;

and

$R_3$ is chloro; fluoro; methyl; t-butyl; $OR_6$, where $R_6$ is methyl or ethyl; or a group

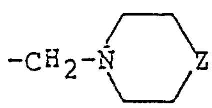,

where

Z is as defined above;

with the provisos that

1) when R is hydrogen, then X is in the 3′ or 4′ position, and when R is chloro, then X is in the 4′ position,

2) when $R_3$ is chloro, fluoro or methyl, then p is other than 3,

3) when $R_3$ is t-butyl or a group

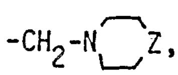

then p is 1, and

4) when p is 2, then the two $R_3$'s are not simultaneously in the 2″ and 6″ positions,

in free form or in acid addition salt form where such salts exist.

2. A compound according to claim 1 of formula Is

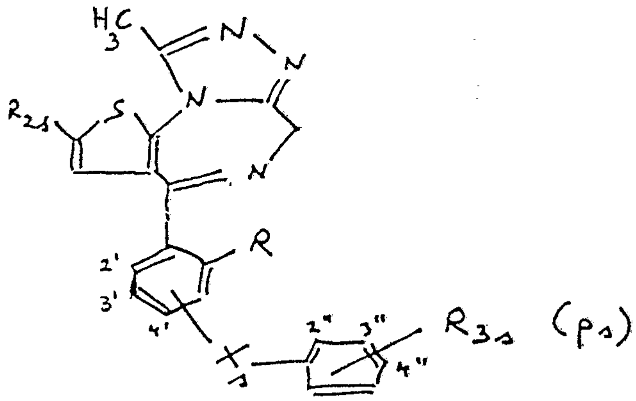

Is

wherein
R is as defined in claim 1,
$R_{2s}$ is hydrogen; a group of the formula $-(CH_2)_mCOOR_4$ wherein m and $R_4$ are as defined in claim 1; or a group of the formula

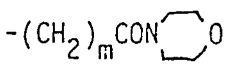

wherein m is as defined in claim 1,
$X_s$ is in the 3′ or 4′ position and is $-(CH_2)_m-$ wherein m is as defined in claim 1,
$p_s$ is 3, and
$R_{3s}$ is $-OR_6$ wherein $R_6$ is as defined in claim 1,
in free form or in acid addition salt form where such salts exist.

3. A compound according to claim 1 of formula I
wherein the substituents are as defined in claim 1 with the further proviso that $R_4$ is other than an alkali metal cation,
in free form or in acid addition salt form where such salts exist.

4. The compound according to claim 1 wherein R is hydrogen, $R_1$ is methyl, $R_3$ is methoxy in the 3″, 4″ and 5″ positions, X is $-(CH_2)_2-$ in the 4′ position and $R_2$ is hydrogen, in free form or in acid addition salt form where such salts exist.

5. A compound according to claim 1 wherein either R, $R_1$, $R_3$ and X are as defined in claim 4 and $R_2$ is $-(CH_2)_2COOCH_3$,

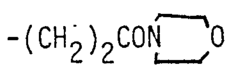

or $-(CH_2)_2COONa$, or R is chloro, $R_1$ is methyl, $R_2$ is hydrogen, $R_3$ is methoxy in the 3″, 4″ and 5″ positions and X is $-(CH_2)_2-$ in the 4′ position, in free form or in acid addition salt form where such salts exist.

6. A pharmaceutical composition comprising a compound according to any one of claims 1 to 5 in free form or in pharmaceutically acceptable salt form where such salts exist.

7. A compound according to any one of claims 1 to 5 for use as a pharmaceutical.

8. A compound according to any one of claims 1 to 5 for use as a PAF inhibitor.

9. A process for the preparation of a compound of formula Iwherein R, $R_1$, $R_3$ and X are as defined in claim 1 and $R_2$ is hydrogen; methyl; ethyl; a group of the formula $-(CH_2)_mCOOR_4$ wherein $R_4$ is methyl or ethyl and m is as defined in claim 1; or a group of the formula $-(CH_2)_mCH_2N(R_5)_2$ wherein m and $R_5$ are as defined in claim 1, which comprises
reacting a compound of the formula A

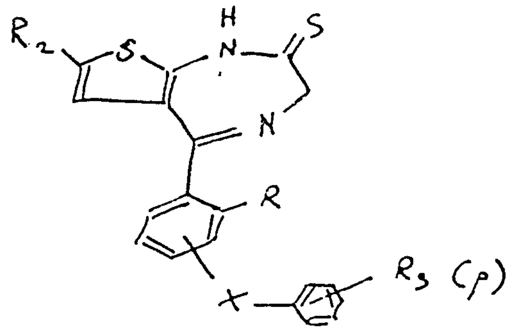

A

wherein R, $R_3$, p and X are as defined in claim 1 and $R_2$ is as defined in claim 1 with the proviso that it is other than a group of the formula $-(CH_2)_mCON(R_5)_2$ wherein m and $R_5$ are as defined in claim 1, with a corresponding compound of the formula AA

$R_1$-CONH-$H_2$ AA

wherein $R_1$ is as defined in claim 1

and recovering the resultant compound of the formula I in free form or in acid addition salt form where such salts exist.

10. A process for the preparation of a compound of the formula I wherein R, $R_1$, $R_3$, p and X are as defined in claim 1 and $R_2$ is a group of the formula $-(CH_2)_mCON(R_5)_2$ wherein m and $R_5$ are as defined in claim 1, which comprises

reacting a corresponding compound of the formula I wherein $R_2$ is a group of the formula $-(CH_2)_mCOOR_4$ wherein m is as defined in claim 1 and $R_4$ is methyl or ethyl,

with a corresponding compound of formula B

$HN(R_5)_2$ B

wherein $R_5$ is as defined in claim 1,

and recovering the resultant compound of formula I in free form or in acid addition salt form where such salts exist.

11. A process for the preparation of a compound of the formula I wherein p, R, $R_1$, $R_3$,and X are as defined in claim 1 and $R_2$ is a group of the formula $-(CH_2)_mCOOR_4$ wherein m is as defined in claim 1 and $R_4$ is an alkali metal cation, which comprises

hydrolysing a corresponding compound of the formula I wherein $R_2$ is a group of the formula $-(CH_2)_mCOOR_4$ wherein $R_4$ is methyl or ethyl,

and recovering the resultant compound of the formula I in free form or in acid addition salt form where such salts exist.

12. A method for the preparation of a pharmaceutical composition which comprises mixing a compound of the formula I as defined in claim 1 in free form or in pharmaceutically acceptable acid addition salt form where such salts exist, with a pharmaceutically acceptable carrier or diluent.

13. Use of a compound of formula I as defined in claim 1 in free form or in pharmaceutically acceptable acid addition salt form where such salts exist, for the preparation of a pharmaceutical composition according to the method of claim 12.

**Claims for the following Contracting States: ES,GR**

1. A process for the preparation of a compound of formula I

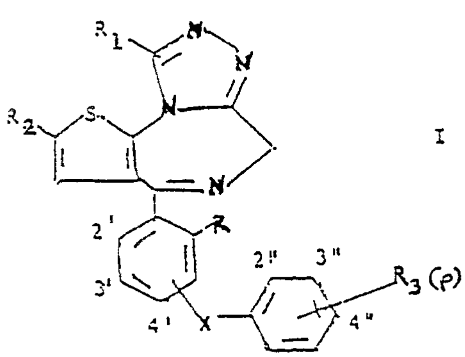

I

where

R is hydrogen or chloro;

$R_1$ is hydrogen; methyl or cyclopropyl;

$R_2$ is hydrogen; methyl; ethyl; a group of the formula

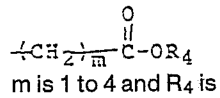

, where

m is 1 to 4 and $R_4$ is methyl or ethyl; a group of the formula

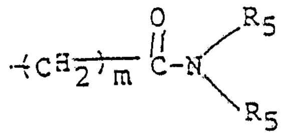

,

where

m is as defined above and each $R_5$, independently, is straight or branched chain $C_{1-3}$alkyl, or the two $R_5$'s together with the nitrogen atom to which they are attached form a group of the formula

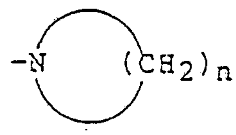

where n

is 4 or 5, or a group of the formula

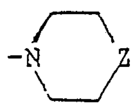

,

where Z is

-O-, -S- or $-NCH_3-$; or a group of the formula

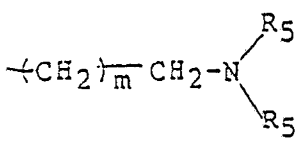

,

where

m and the $R_5$'s are as defined above;

X is $-(CH_2)_{\overline{m}}$ where m is as defined above; $-OCH_2-$ or $-CH_2OCH_2-$;

p is 0 or an integer 1 to 3;

and

$R_3$ is chloro; fluoro; methyl; t-butyl; $OR_6$, where $R_6$ is methyl or ethyl; or a group

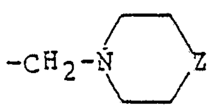

,

where

Z is as defined above;

with the provisos that

1) when R is hydrogen, then X is in the 3′ or 4′ position, and when R is chloro, then X is in the 4′ position,

2) when $R_3$ is chloro, fluoro or methyl, then p is other than 3,

3) when $R_3$ is t-butyl or a group

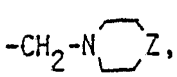

then p is 1, and

4) when p is 2, then the two $R_3$'s are not simultaneously in the 2″ and 6″ positions,

in free form or in acid addition salt form where such salts exist,

which comprises

reacting a compound of the formula A

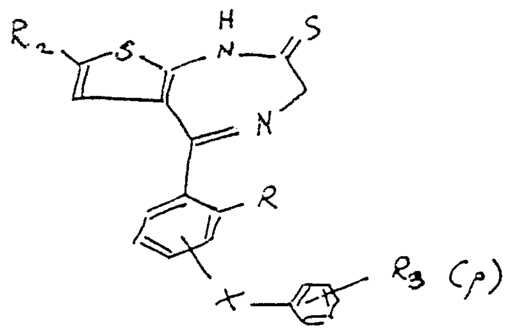

A

wherein R, $R_2$, $R_3$, p and X are as defined above,
with a corresponding compound of the formula AA
$R_1$-CONH-$NH_2$ AA
wherein $R_1$ is as defined above,
and recovering the resultant compound of formula I in free form or in acid addition salt form where such salts exist.

2. A process according to claim 1 for the preparation of the compound of formula I wherein R is hydrogen, $R_1$ is methyl, $R_2$ is hydrogen, $R_3$ is methoxy in the 3″, 4″ and 5″ positions and X is $-(CH_2)_2-$ in the 4′ position, in free form or in acid addition salt form where such salts exist.

3. A process according to claim 1 for the preparation of the compounds of formula I wherein $R_1$, $R_3$ and X are as defined in claim 2 and either R is hydrogen and $R_2$ is $-(CH_2)_2COOCH_3$, or R is chloro and $R_2$ is hydrogen, in free form or in acid addition salt form where such salts exist.

4. A process for the preparation of a compound of the formula I wherein R, $R_1$, $R_3$, p and X are as defined in claim1 and $R_2$ is a group of the formula $-(CH_2)_mCON(R_5)_2$ wherein m and $R_5$ are as defined in claim 1,
which comprises reacting a corresponding compound of the formula I wherein $R_2$ is a group of the formula $-(CH_2)_mCOOR_4$ wherein m and $R_4$ are as defined in claim 1,
with a corresponding compound of the formula B
$HN(R_5)_2$ B
wherein $R_5$ is as defined in claim 1,
and recovering the resultant compound of formula I in free form or in acid addition salt form where such salts exist.

5. A process according to claim 4 for the preparation of the compound of formula I wherein R, $R_1$, $R_3$ and X are as defined in claim 2 and $R_2$ is

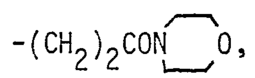

in free form or in acid addition salt form where such salts exist.

6. A process for the preparation of a compound of the formula I wherein R, $R_1$, $R_3$, p and X are as defined in claim 1 and $R_2$ is a group of the formula $-(CH_2)_mCOOR_4$ wherein m is as defined in claim 1 and $R_4$ is an alkali metal cation, which comprises
hydrolysing a corresponding compound of the formula I wherein $R_2$ is a group of the formula $-(CH_2)_mCOOR_4$ wherein $R_4$ is methyl or ethyl, and m is as defined in claim 1, and recovering the resultant compound of the formula I in free form or in acid addition salt form where such salts exist.

7. A process according to claim 6 for the preparation of the compound of formula I wherin R, $R_1$, $R_3$ and X are as defined in claim 2 and $R_2$ is $-(CH_2)_2COONa$, in free form or in acid addition salt form where such salts exist.

8. A method for the preparation of a pharmaceutical composition which comprises mixing a compound of the formula I as defined in any one of claims 1 to 7 in free form or in pharmaceutically acceptable acid addition salt form where such salts exist, with a pharmaceutically acceptable carrier or diluent.

European Patent Office

# EUROPEAN SEARCH REPORT

Application Number

EP 89 81 0291

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP-A-0 230 942 (BOEHRINGER INGELHEIM KG)<br>* claims 1,4,8-10,13; table 6 * | 1,6-8, 11-13 | C 07 D 495/14<br>A 61 K 31/55 //<br>(C 07 D 495/14<br>C 07 D 333:00<br>C 07 D 249:00<br>C 07 D 243:00 ) |
| A | EP-A-0 194 416 (BOEHRINGER INGELHEIM INTERNATIONAL GMBH)<br>* claims 1,2,7-9,12; pages 29-33 * | 1,6-8, 11-13 | |
| A | DE-A-3 624 779 (BOEHRINGER INGELHEIM KG)<br>* claim 1; page 5, reaction scheme; page 6, lines 27-31 * | 1,6-10 | |
| A | EP-A-0 240 899 (BOEHRINGER INGELHEIM KG)<br>* claims 1,8-10; examples 43,44,47,48 * | 1,6-8 | |
| D,A | WO-A-8 800 587 (SANDOZ AG)<br>* abstract * | 8 | |

TECHNICAL FIELDS SEARCHED (Int. Cl.4)

C 07 D 495/00

The present search report has been drawn up for all claims

| Place of search | Date of completion of the search | Examiner |
|---|---|---|
| BERLIN | 12-07-1989 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)